Europäisches Patentamt

European Patent Office

Office européen des brevets

(11) Publication number: **0 475 553 A2**

# EUROPEAN PATENT APPLICATION

(21) Application number: **91300664.9**

(22) Date of filing: **29.01.91**

(51) Int. Cl.5: **C07K 15/14**

(30) Priority: **10.09.90 JP 237187/90**

(43) Date of publication of application:
**18.03.92 Bulletin 92/12**

(84) Designated Contracting States:
**DE FR GB**

(71) Applicant: **SMOKING RESEARCH FOUNDATION**
**18th, Mori-Bldg, 3-13, Toranomon 2-chome**
**Minato-ku, Tokyo, 105(JP)**

(72) Inventor: **Hasekura, Hayato**
**7-11, Sawamura 1-chome**
**Matsumoto-shi, Nagano(JP)**
Inventor: **Yonemura, Isamu**
**3-5-22, Kiri**
**Matsumoto-shi, Nagano(JP)**
Inventor: **Okano, Akira**
**2-8-27, Miyabuchi**
**Matsumoto-shi, Nagano(JP)**
Inventor: **Shimizu, Yoshiharu**
**3-1207-15, Sayama**
**Higashiyamato-shi, Tokyo(JP)**

(74) Representative: **Thomson, Paul Anthony et al**
**Potts, Kerr & Co. 15, Hamilton Square**
**Birkenhead Merseyside L41 6BR(GB)**

(54) **Life span determining protein and method of isolating the same.**

(57) A protein which determines the life spun. The protein is produced within a living body at an early stage of development to have close interrelation with the life span determined at this stage. It is estimated that the protein is an expression product of the longevity gene. The protein has a molecular weight of about 77,000, an isoelectric point of about pH 6.5, a molecular distinction coefficient

$$A_{280}^{1\%}$$

of about 18, a hexose content of about 3.3%. It exhibits the life span prolonging effect when daily dosed to an adult with feeds or foods.

## BACKGROUND OF THE INVENTION:

### Field of the Invention;

The present invention relates to a life span determining protein and method of isolating the same.

### Related Art Statement;

To explain the mechanism of ageing, there have been two prominent theories: the program theory that postulates genes controlling senility which may express abnormal proteins as the ageing proceeds, and the somatic mutation theory claiming that some abnormality occurs in composition or electric charge of proteins when the accumulation of gene injuries by somatic mutation reaches a threshold (Hayflick, Exp. Gerontol., 20, 145 - 159 (1985)). Either of these theories predicts some protein changes will occur at the later period of life. In view of such prediction, many investigations have been made to find out significant change of proteins.

However, any significant change of proteins at the later period of life has not yet been found even by referring to various investigations.

For example, Vaughan et al. investigated brain proteins of mice to find out any change. They found significant difference between the proteins of fetal mice and the proteins of juvenile mice, but no significant difference has been found between the proteins of young mice and the proteins of aged mice (Gerontology, 23, 110 - 126, (1977)). Wilson et al. examined proteins of nerve tissue of rats by two-dimensional electrophoresis, and reported that no novel protein was detected from the proteins of aged rats (Gerontology, 24, 426 - 433, (1978)). Parker et al. compared the proteins from young Drosophila melanogaster at the age of 6 days post emergence with the proteins from old Drosophila melanogaster at the age of 60 days post emergence by two-dimensional electrophoresis, but failed to find any significant difference (Mech. Age. Dev., 16, 127 - 139, (1981)). In view of the results of these investigations, many researchers have reached the conclusion that either of the program theory and the somatic mutation theory should not be acceptable. The only exception is the report by Fleming et al. who reported that characteristic seven small protein spots were found on the electrophoresis pattern of two-dimensional electrophoresis of proteins extracted from Drosophila melanogaster aged 28 days when the whole proteins of Drosophila melanogaster on the days 10, 28 and 44 were compared with one another (Science, 231, 1157 -1159, (1986)). However, they failed to clarify whether such proteins caused the senescence or were produced as a result of senescence.

On the other hand, results of genetic studies on Drosophila melanogaster suggest the presence of longevity gene rather than the presence of a senescence gene. We have investigated on the life spans of highly purebred strains of Drosophila melanogaster by successive sub-mating over thirteen generations, and found that the life span is controlled by major genes (Yonemura et al., Proc. Japan Acad., 62, Ser. B, 41 - 44, (1986)). In this connection, Luckinbill et al, reported that the major genes controlling the life span of Drosophila melanogaster were located on chromosomes 1 and 3 (Heredity, 60, 367 - 374, (1988)). We also discovered an autosomal major gene and a sex chromosomal major gene which controlled the life span of Drosophila melanogaster, these genes being transmitted by Mendelian inheritance and nominated as Jm (Ju-myo) genes (Yonemura et al., Hereditas, 111, 207 - 214, (1989); Yonemura et al., Hereditas, 112, 117 - 127, (1990)).

We have further analyzed the interrelation between the adult life span of Drosophila melanogaster and the hatching time (the time between egg-laying and hatching) and the emerging time (the time between egg-laying and emerging) to find that the Jm genes influence the preimaginal developmental speed in the late larval stage to emergence and that the maximum adult life span is determined already at the emergence stage (Yonemura et al., Heredity, 66, (1990) (in press)). If the Jm genes express themselves in the last half of the late larval stage to emergence, trace of the expression of the Jm genes should be found in the adult immediately after the emergence. Bearing in mind such a view point, we have studied the periodical change in whole proteins between the long-lived and short-lived purebred strains from the larval stage to the senile death. The study revealed that a protein having a molecular weight of about 77,000 was found distinctively at the time immediately after the emergence, and that there is a possibility that the protein was a product of expression of Jm genes and determines the life span (Okano et al., Biomed. Gerontol., 13(2), 137-138, (1989)).

We have isolated and refined the protein having a molecular weight of about 77,000 and dosed the same to adult Drosophila melanogaster to find that the life span has been considerably prolonged by the dosage of the protein.

Accordingly, the present invention has been accomplished on the basis of the finding described above to provide a novel medical drug, healthy or functional foods, an additive to feed stuff and a novel reagent for various research works.

OBJECT AND SUMMARY OF THE INVENTION:

An object of this invention is to provide a protein which determines the life span of animals.

Another object of this invention is to provide a method of isolating a protein which determines the life span of animals.

The present invention provides a protein which determines the life span of animals. The protein provided by this invention has a molecular weight of about 77,000, an isoelectric point of about pH 6.5, a hexose content of about 3.3% and a molecular extinction coefficient

$$A_{280}^{1\%}$$

of about 18.

The protein has the following amino acid composition (in mol%) of:

| Asx (Asp + Asn) | 10.5 |
| Glx (Glu + Gln) | 11.0 |
| Ser | 5.6 |
| Gly | 7.2 |
| His | 7.6 |
| Arg | 3.5 |
| Thr | 3.3 |
| Ala | 5.0 |
| Pro | 4.4 |
| Tyr | 8.3 |
| Val | 7.9 |
| Met | 3.0 |
| Ile | 3.0 |
| Leu | 6.7 |
| Phe | 7.6 |
| Lys | 5.4 |
| (Trp and Cys have not been examined.) | |

The protein of the invention is adsorbed on the Con-A (Concanavalin A) column and is eluted with α-methyl-D-mannoside, and thus it is estimated that the protein is a glycoprotein having the mannose core.

The content of the protein of the invention is appreciably different between the whole protein of the short-lived Drosophila melanogaster strain and the whole protein of the long-lived Drosophila melanogaster strain, and the ratio of the protein of the invention in the whole protein of the long-lived Drosophila melanogaster is appreciably high. In particular, the content of the protein is the highest in the early half time of the pupal stage. It is thus estimated that the protein of the invention has some connection with the life span which is determined at that stage, since the content thereof is larger in the long-lived strain and it is produced in a large quantity at the early time of the pupal stage.

The protein of the invention is intensely adsorbed by the DEAE-Sephacel and eluted by an eluent having a high ionic strength. It is also adsorbed by the Con-A column and eluted by highly concentrated α-methyl-D-mannoside solution. Accordingly, the protein of the invention may be isolated and refined by subjecting a starting material (for example, the supernatant of homogenized early stage pupae of Drosophila malanogaster) to anion exchange column chromatography using the DEAE-Sephacel followed by Con-A column chromatography. Gel filtration may also be used when desired.

BRIEF DESCRIPTION OF THE DRAWINGS:

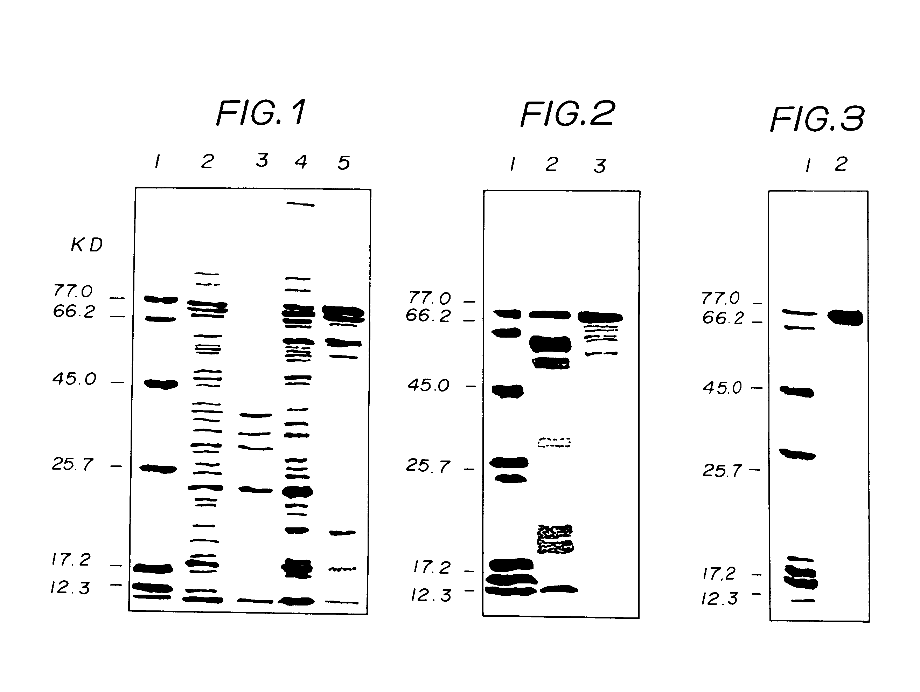

Fig. 1 shows SDS-polyacrylamide gel electrophoresis charts of the fractions obtained through DEAE-Sephacel column chromatography; wherein Lane 1 is molecular weight markers; Lane 2 is the whole

EP 0 475 553 A2

proteins of Drosophila melanogaster pupae extracted with PBS and applied to the column; Lane 3 is Fraction 1 eluted with a 50 mM Tris-HCl buffer solution; Lane 4 is Fraction 2 eluted with a 450 mM Tris-HCl buffer solution; and Lane 5 is Fraction 3 eluted with a 500 mM Tris-HCl buffer solution containing 0.5 M NaCl.

Fig. 2 shows SDS-polyacrylamide gel electrophoresis charts of the fractions obtained through Con-A Sepharose column chromatography; wherein Lane 1 is molecular weight markers; Lane 2 is Fraction 1 eluted with a 20 mM Tris-HCl buffer solution (pH 7.2) containing 0.5 M NaCl; and Lane 3 is Fraction 2 eluted with a 20 mM Tris-HCl buffer solution containing 0.2M α-methyl-D-mannoside and 0.5 M NaCl.

Fig. 3 shows the fraction pattern of the refining gel filtration with the Sephadex G-200.

Fig. 4 shows SDS-polyacrylamide gel electrophoresis charts of the fraction obtained through the refining gel filtration with Sephadex G-200; wherein Lane 1 is molecular weight markers; and Lane 2 is the fraction obtained through the refining gel filtration.

Fig. 5 shows the two-dimensional electrophoresis pattern of the protein of the invention. The protein is subjected to an isoelectric focusing along the abscissa and then subjected to SDS-polyacrylamide electrophoresis along the ordinate.

Fig. 6 shows the ultraviolet absorption spectrum of the protein of the invention.

Fig. 7 is a graph showing the life prolongation effect of the protein of the invention when it is fed to a short-lived Drosophila melanogaster strain.

Fig. 8 is a graph showing the life prolongation effect of the protein of the invention when it is fed to a long-lived Drosophila melanogaster strain.

## EMBODIMENT OF THE INVENTION:

Presently preferred embodiments of the invention will now be described by referring to some examples thereof. However, it is to be noted that the invention should not be limited only to the following examples.

## EXAMPLE 1

About 20,000 (23.5g) bodies of early stage pupae (up to 2 days post pupation) of the long-lived Drosophila melanogaster strain were homogenized in 100 ml of ice-cooled PBS (20 mM phosphate buffer solution containing 0.15 M NaCl, pH 7.2), and stirred at 4°C overnight to extract proteins. The homogenized suspension was subjected to centrifugal separation at 100,000 x G for 2 hours to separate the supernatant. The precipitate was subjected again to extraction using 100 ml of ice-cooled PBS, and then the supernatant was separated in a simlar manner as aforementioned and added to the supernatant obtained by the first extraction. The thus collected supernatants were subjected to ultrafiltration using Amicon Diaflow Membrane PM-30 to obtain about 10 ml of a concentrated solution (containing 3.9 grams of proteins; Yield: 16.7%).

The thus obtained concentrated solution was dialyzed against a 50 mM Tris-HCl buffer solution (pH 8.2), and then applied on a DEAE-Sephacel column (1.6φ x 25 cm). The column was stepwise eluted with 200 ml of a 50 mM Tris-HCl buffer solution (pH 8.2), 300 ml of a 450 mM Tris-HCl buffer solution (pH 8.2) and 200 ml of a 500 mM Tris-HCl buffer solution (pH 8.2) containig 0.5 M NaCl. Fractions of respective eluted solutions showing an absorption peak at 278 nm were separated and named Fractions 1, 2 and 3. Each fraction was dialyzed against PBS, and then concentrated by ultrafiltration using Amicon Diaflow Membrane PM-30. Concentrated fractions were subjected to SDS-polyacryamide gel-electrophoresis. The results are shown in Fig. 1. As shown, Fraction 1 eluted with 50 mM Tris-HCl buffer solution does not contain the protein having a molecular weight of about 77,000 (Lane 3). Most of the contaminating proteins are contained in Fraction 2 eluted with 450 mM Tris-HCl buffer solution (Lane 4). Major portion of the objective protein having a molecular weight of about 77,000 was found in Fraction 3 eluted with the 500 mM Tris-HCl buffer solution containing 0.5 M NaCl, and the major part of whole protein in Fraction 3 was held by that protein (as shown by Lane 5). Meanwhile, Lane 1 shows the electrophoresis pattern of molecular weight markers, and Lane 2 shows the electrophoresis pattern of whole proteins of PBS extraction from Drosophila melanogaster.

The volume of concentrated Fraction 3 was 3 ml, and thus the weight of the ojective protein was about 0.021 gram and the yiled was 0.089%.

The concentrated Fraction 3 was dialyzed against a 20 mM Tris-HCl buffer solution (pH 7.2) containing 0.5 M NaCl, and then applied on a Con-A Sepharose column equilibrated with the same buffer solution. Fraction 1 was separated by eluting the column with 100 ml of the same buffer solution, and Fraction 2 was separated by eluting with a 20 mM Tris-HCl buffer solution (pH 7.2) containing 0.5 M NaCl and 0.2 M α-methyl-D-mannoside. Each fraction was dialyzed against PBS and then concentrated by ultrafiltration through Amicon Diaflow Membrane PM-30, and thereafter subjected to SDS-polyacrylamide electrophoresis. As shown in Fig. 2, Fraction 2 (Lane 2) contained only a small amount of the objective protein having a molecular weight of about 77,000, whereas Fraction 3 (Lane 3) contained a large amount of the protein. Lane 1 represents molecular weight markers.

The protein of the invention binds to Con-A, and thus it is estimated that the protein is a glycoprotein having sugar chains(s) containing mannose core.

The volume of concentrated Fraction 2 was 2 ml, and the weight of proteins was 0.011 gram and the yield was 0.046%.

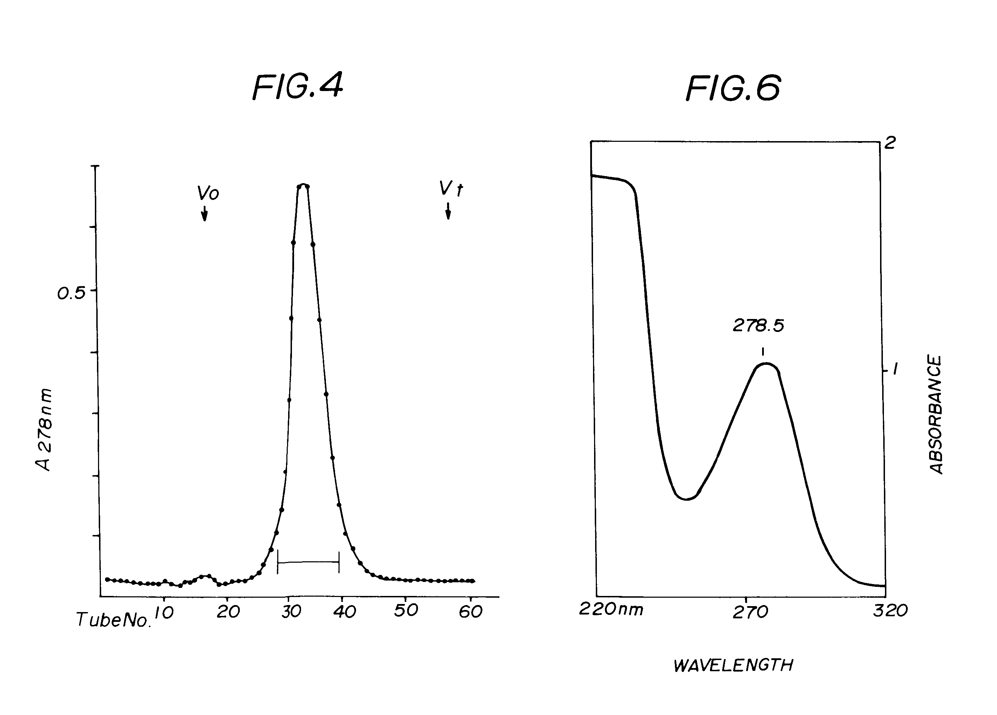

Fraction 2 from the Con-A column was further refined through gel filtration. In detail, concentrated Fraction 2 was applied on a Sephadex G-200 column (super fine, 1.5$\phi$ x 98 cm) equilibrated with PBS, and eluted with the same solution. The fore 2/3 part of the peak of the eluant (Fraction Nos. 30 - 37) was collected, and subjected again to gel filtration using the same column. A substantially symmetrical peak was observed (see Fig. 3). Fraction Nos. 28 to 40 shown in Fig. 3 were collected and subjected to SDS-polyacrylamide electrophoresis to find that a substantially single band was observed and the band was corresponding to a molecular weight of about 76,600 (see Lane 2 in Fig. 4; Lane 1 shows the electrophoresis pattern of molecular weight markers. The protein of the invention was obtained in a final quantity of 0.005 gram, and the total yield was 0.021%.

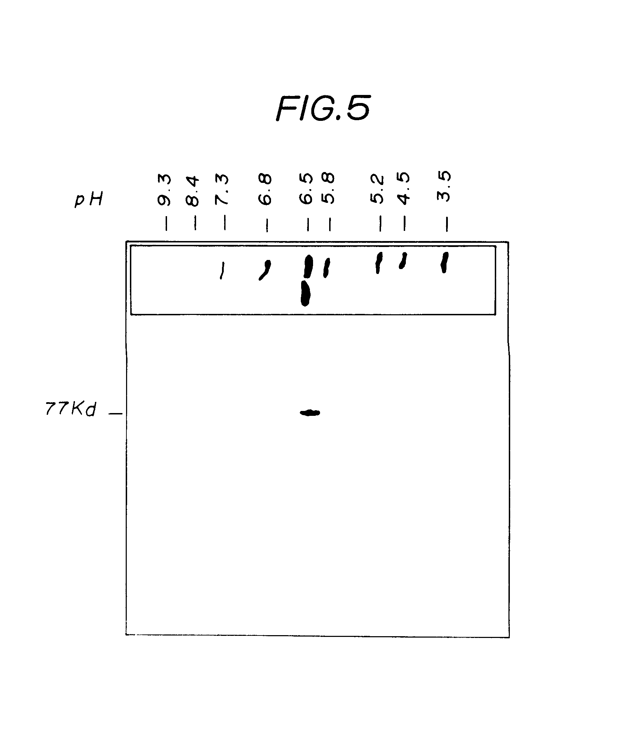

The thus isolated protein was subjected to two-dimensional electrophoresis. Initially, using IEF PAGE MINI pH 3-10 (Produced by TEFCO Co.), the protein was subjected to isoelectric focusing for an hour while applying an electric potential of 100 volts per one gel plate (80x80x1 mm), for additional one hour while applying an electric potential of 200 volts, and for 30 minutes while applying an electric potential of 300 volts. The lane containing the electrofocusing band was cut off and immersed in an SDS solution (125 mM Tris-HCl, pH 6.8, 2% mercaptoethanol, 2% SDS, 2% glycerol and BPB) for 3 minutes, and then the lane was put on an SDS-polyacrylamide gel to be subjected to electrophoresis along the second direction. The result is shown in Fig. 5. The isoelectric point of the protein was pH 6.5.

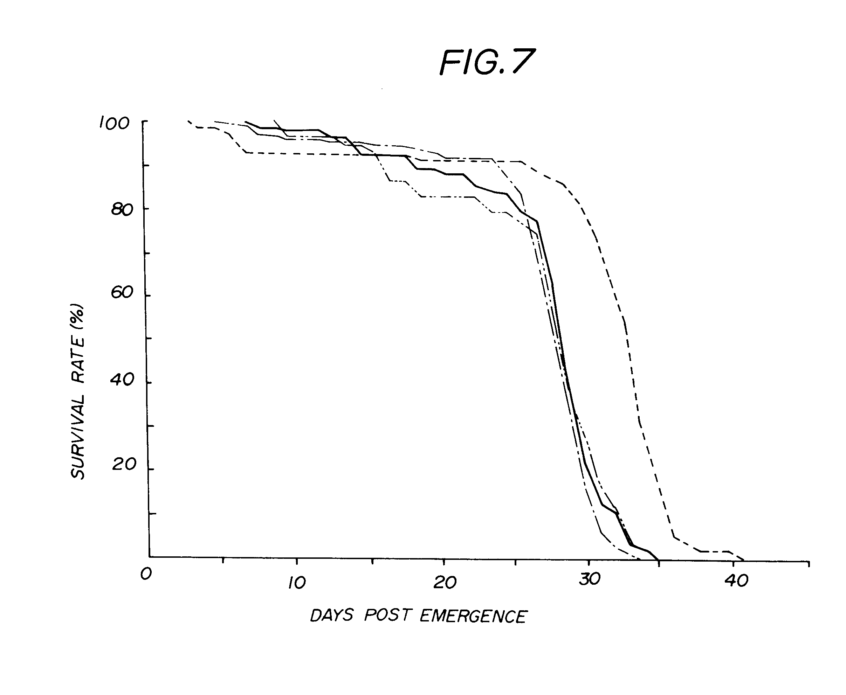

Fig. 6 is the ultraviolet absorption spectrogram of the solution of the refined protein of the invention. The molecular extention coefficient of the protein of the invention was determined. The result revealed that

$$A^{1\%}_{280} = 18.3$$

The content of hexose in the protein of the invention was determined by the phenol sulfuric acid method (Anal. Biochem., 147, 222 - 229, (1985)) to find that the hexose content was about 3.3%.

The amino acid composition (mol%) of the protein was as follows.

| | |
|---|---|
| Asx (Asp + Asn) | 10.5 |
| Glx (Glu + Gln) | 11.0 |
| Ser | 5.6 |
| Gly | 7.2 |
| His | 7.6 |
| Arg | 3.5 |
| Thr | 3.3 |
| Ala | 5.0 |
| Pro | 4.4 |
| Tyr | 8.3 |
| Val | 7.9 |
| Met | 3.0 |
| Ile | 3.0 |
| Leu | 6.7 |
| Phe | 7.6 |
| Lys | 5.4 |
| (Trp and Cys were not examined) | |

EXAMPLE 2

The refined protein of the invention was fed to adult <u>Drosophila melanogaster</u> by adding the same to the feed to examine the influence thereof on the adult life span of <u>Drosophila melanogaster</u>.

The composition of the feed given to the adult <u>Drosophila melanogaster</u> was as follows.

| Dry Yeast | 8% |
|---|---|
| Sugar | 5% |
| Agar | 1% |
| Propionic Acid | 0.5% |
| n-Butyl p-Hydroxybenzoate | 0.5% |
| Water | 85% |

12.5 $\mu$l of a 1/4 diluted PBS solution containing 0.02 $\mu$g/ml of the protein of the invention was added to 0.5 ml the feed set forth above, and 58 male adults of short-lived <u>Drosophila melanogaster</u> strain was fed with the feed added with the protein of the invention from 5 days post emergence. For comparison purpose, Control 1 (86 male adults) was fed with the feed which was not added with the protein of the invention, Control 2 (60 male adults) was fed with the feed added with 12.5 $\mu$l of 1/4 diluted PBS, and Control 3 (235 male adults) was fed with the feed which was added with 12.5 $\mu$l of a 1/4 diluted PBS containing 0.02 $\mu$g/ml of Fraction 3 passing through the DEAE-Sephacel column unadsorbed in Example 1.

Dead individuals were removed from respective groups every day, while living individuals in respective groups were fed with fresh feeds, and the periodical changes in survival rate of respective groups were examined. The results are shown in Fig. 7. The numbers of dead individuals was abruptly increased in Cotrol 1 (shown by the real line), Control 2 (shown by the double dots-and-dash line) and Control 3 (the single dot-and-dash line) from 27 days, the longest life span in Control 3 being 34 days and the longest life spans in Controls 1 and 2 being 35 days. In general, no significant difference was found among the Control groups. In contrast thereto, in the group (shown by the broken line) dosed with the glycoprotein of the invention, most of the flies survived longer than 30 days, and the longest life span was 41 days. When compared to the control groups, the average life prolongation effect was about 5 days. The life prolongation effect as such was significant.

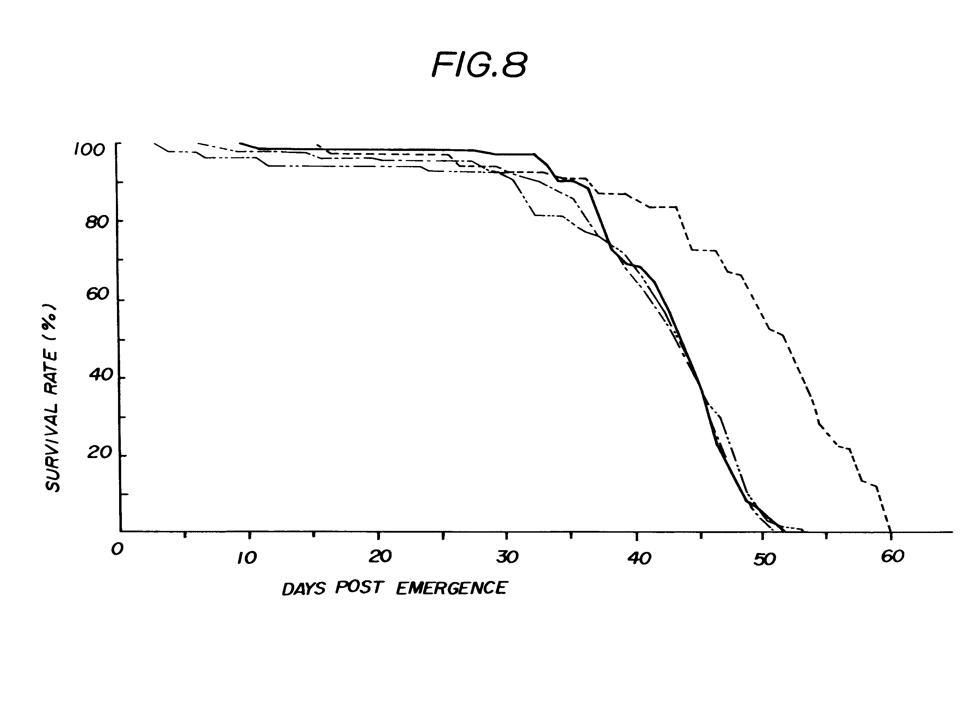

Same experience was conducted while using male flies of long-lived Drosophila melanogaster strain to reveal that utterly a same result was obtained. As shown in Fig. 8, numbers of dead individuals abruptly increased from 37 days in Control 1 (87 flies, shown by the real line), Control 2 (60 flies, shown by the dots-and-dash line) and in Control 3 (273 flies, shown by the dot-and-dash line), the longest life spans in Controls 1 and 3 being 52 days and the longest life span in Control 2 being 54 days, and no significant difference was observed between these Control groups. In contrast thereto, in the group (57 flies, shown by the broken line) dosed with the protein of the invention, the number of dead individuals increased after the lapse of 45 days and the longest life span was 60 days. The result showed that the average life span was prolonged by about 7 to 8 days, as compared to the Control groups. As will be appreciated from the results of the experiences, the present invention provides a novel protein which exerts life prolongation effect on both of the short-lived and long-lived strains.

As will be clearly understood from the foregoing, the protein provided by the present invention exerts appreciable life prolongation effect and it is estimated that the protein of the invention is a novel protein which determines the life span. Accordingly, the protein of the invention is expected to be used as a novel additive to be added to foodstaffs including healthy foods and to feeds for animals, and also to be used as a medicine.

The protein of the invention may be used as a useful reagent which is used in research works for investigating the life span, since it is most likely that the protein of the invention has some connection with the longevity gene.

## Claims

1. A protein determing the life span.

2. The protein of claim 1, wherein said protein has a molecular weight of about 77,000, an isoelectric point of about pH 6.5, a hexose content of about 3.3% and a molecular extinction coefficient

$$A_{280}^{1\%}$$

of about 18.

3.  The protein of claim 1, wherein said protein is extracted from Drosophila melanogaster.

4.  The protein of claim 1, wherein said protein is prepared by a process comprising the steps of using the whole proteins of pupal Drosophila melanogaster as a starting material, and isolating a protein having a molecular weight of about 77,000 by ion exchange chromatography and subsequent Con-A affinity chromatography.

5.  A process for isolating a protein which determines the life span, comprising the steps of using the whole proteins of pupal Drosophila melanogaster as a starting material, and isolating a protein having a molecular weight of about 77,000 by ion exchange chromatography and subsequent Con-A affinity chromatography.

FIG.3

FIG.2

FIG.1

FIG.4

FIG.6

# FIG.5

FIG.7

EP 0 475 553 A2

FIG.8

SURVIVAL RATE (%)

DAYS POST EMERGENCE

12